# EUROPÄISCHE PATENTSCHRIFT

(11) **EP 0 716 305 B1**
(45) Veröffentlichungstag und Bekanntmachung des Hinweises auf die Patenterteilung: **29.09.1999**
(21) Anmeldenummer: 95118820.0
(22) Anmeldetag: 30.11.1995
(51) Int. Cl.: G01N 33/543, G01N 33/76

(54) **Vorrichtung zur Durchführung eines Schwangerschaftstests**
Device to execute a pregnancy test
Dispositif pour exécuter une épreuve de grossesse

(30) Priorität: 06.12.1994 DE 4443386
(43) Veröffentlichungstag der Anmeldung: 12.06.1996
(73) Patentinhaber: SERATEC Gesellschaft für Biotechnologie mbH, 37079 Göttingen (DE)
(72) Erfinder: Mast, Wolf-Peter, Dr., D-29320 Hermansburg (DE)
(74) Vertreter: Rehberg, Elmar, Dipl.-Ing.

(56) Entgegenhaltungen:
- EP-A- 0 291 194
- EP-A- 0 299 428
- EP-A- 0 362 809
- EP-A- 0 383 619

## Beschreibung

Die Erfindung betrifft eine Vorrichtung zur Durchführung eines Schwangerschaftstests zur Feststellung des Hormons HCG im Urin, mit einem Teststreifen, der einen Träger mit einem Benetzungsvorsprung aufweist und auf dem Urin weiterleitendes Material sowie Antikörper zur Anzeige entsprechender Reaktionen untergebracht sind, wobei der Teststreifen mit dem bei Benutzung zumindest teilweise überstehenden Benetzungsvorsprung in einem Gehäuse angeordnet ist. Statt einen gesonderten Teststreifen mit Träger in das Gehäuse einzusetzen, kann das Gehäuse auch direkt als Träger bzw. Teststreifen dienen, also mit dem Urin weiterleitenden Material sowie mit Antikörpern versehen sein. Solche Vorrichtungen dienen zur Durchführung eines Schwangerschaftstestes, der sich schnell, präzise und zuverlässig durchführen läßt. Diese Vorrichtungen nutzen die Tatsache, daß bei Schwangeren am ersten Tag nach Ausbleiben der Regel, also etwa nach 14 Tagen nach einer Konzeption, im Urin der Schwangeren das Hormon HCG feststellbar ist.

Der Aufbau und die Arbeitsweise bekannter Vorrichtungen zur Durchführung eines solchen Schwangerschaftstestes ist prinzipiell gleich, wobei der eigentliche Test meist mit Hilfe eines Teststreifens durchgeführt wird, der oft in einem besonders ausgebildeten Gehäuse untergebracht ist. Der Teststreifen besitzt einen Träger, der meist aus einem Abschnitt einer PE-Folie besteht. Im Mittelbereich dieses Trägers ist eine Membran aufgebracht, die in einem Bereich Antikörper gegen das Hormon HCG sowie weitere Antikörper zur Anzeige eine ordnungsgemäß abgelaufenen Reaktion aufweist. In einem Glasfaserkissen sind Farbstoffe untergebracht. In den beiden Endbereichen ist der Träger mit Abschnitten eines saugenden Belages, meist in Form von Glasfasern, versehen, wobei der Belag am einen Ende sich in den Bereich eines Benetzungsvorsprunges erstreckt, an welchem der zu untersuchende Urin aufzunehmen ist. Durch die saugenden Beläge wird eine Transport- und Fließrichtung auf dem Träger vorgegeben, so daß bei ordnungsgemäßem Ablauf Farbreaktionen auftreten. Es sind grundsätzlich zwei Farbreaktionen vorgesehen, wobei die eine Farbreaktion bei ihrem Auftreten positiv zu erkennen gibt, daß die Vorrichtung nicht beschädigt war, sich in brauchbarem Zustand befand und die Reaktionen ordnungsgemäß abgelaufen sind, während die andere Farbreaktion eine Aussage darüber macht, ob Schwangerschaft vorliegt oder nicht, also das Hormon HCG vorhanden ist oder nicht. Für die Einleitung des Urins in den Teststreifen ist der Benetzungsvorsprung vorgesehen, der für den Fall, daß der Urin in einem Becher oder einem anderen Gefäß aufgefangen wird und die Untersuchung dann durch Eintauchen erfolgt, bis zu einer Markierung eingetaucht wird, damit eine Direktbenetzung der Membran mit der möglichen Folge von Fehlreaktionen vermieden wird. Wird dagegen die Vorrichtung mit ihrem Benetzungsvorsprung durch Direktbenetzung mit dem Urin in Kontakt gebracht, besteht erhöht die Gefahr, daß nicht für die direkte Benetzung vorgesehene Bereiche des Teststreifens mit dem Urin in Kontakt kommen und so Fehlreaktionen und Fehlanzeigen ausgelöst werden.

Wenn die Vorrichtung nur aus einem Teststreifen, also ohne zusätzliches Gehäuse, besteht, so führt eine ordnungsgemäße Anwendung der Vorrichtung zu einem sicheren Ergebnis. Für eine Direktbenetzung ist ein solcher Teststreifen jedoch kaum verwendbar. Solche Teststreifen besitzen nur eine geringe Länge und Breite, so daß sie für ihre Verpackung während des Transportes zur Anwenderin nur einen geringen Raum benötigen. Sie lassen sich weiterhin einfach entsorgen. Zu dem Teststreifen wird eine Gebrauchsanweisung geliefert, also ein Stück Papier, auf dem die erforderlichen Informationen aufgedruckt sind, wobei es erforderlich ist, diese bei der Anwendung genau zu beachten. Dabei geht es z. B. darum, den Benutzungsvorsprung bei der Benetzung nach unten zu halten, den Teststreifen jedoch während des Ablaufes der Reaktion in eine horizontale Ausrichtung zu bringen. Eine falsche Direktbenetzung führt häufig zum Versagen der Vorrichtung bzw. der Anzeige.

Wenn dagegen der Teststreifen in einem Gehäuse aus Kunststoff angeordnet ist, besteht zunächst ein gewisser Schutz für den Teststreifen. Die Gehäuse aus Kunststoff können in Form von Platten oder Stäben ausgebildet sein, wobei auch mehrteilige Ausführungen nach Art eines Schreibgerätes bekannt sind, so daß so der Benetzungsvorsprung mit einer abziehbaren Hülle während des Transportes geschützt ist. Wesentliche Bereiche des Teststreifens sind im Innern des Gehäuses untergebracht, so daß das Gehäuse notwendigerweise ein Fenster aufweisen muß, damit die entsprechenden Reaktionen durch Auftreten von Farbstreifen von außen erkennbar sind. Diese Fenster erbringen bei Direktbenetzung die Gefahr, daß Bereiche des Teststreifens vorzeitig mit Urin in Kontakt kommen und dadurch die Anzeige verfälscht wird. Diese Gehäuse aus Kunststoff haben an sich nur eine Haltefunktion für den Teststreifen. Sie nehmen ansonsten an der Reaktion nicht teil und erschweren die Entsorgung.

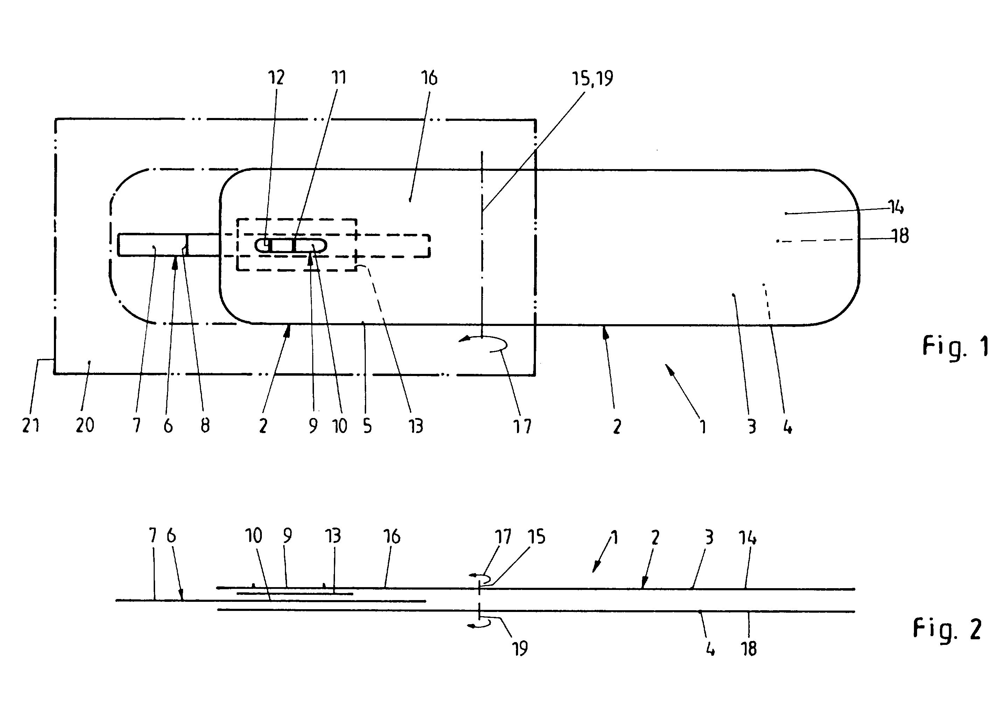

Die EP 0 383 619 A1 zeigt zeigt eine Vorrichtung zur Durchführung eines Schwangerschaftstests zur Feststellung des Hormons HCG im Urin, mit einem Teststreifen, der in einem hohlen Gehäuse untergebracht ist. Das Gehäuse besitzt eine Öffnung, damit der in dem Gehäuse neben dem als separates Teil ausgebildeten Teststreifen untergebrachte Benetzungskörper mit dem Urin in Kontakt kommen kann. Dem Teststreifen ist also ein gesonderter Benetzungskörper zugeordnet, der die Aufgabe hat, sicherzustellen, daß eine gewisse Menge Urin dem Teststreifen zugeführt wird. Der teilweise aus dem hohlen Gehäuse herausschauende, also überstehend vorgesehe Benetzungskörper ist in der Nicht-Gebrauchsstellung durch eine Kappe abgedeckt ist. Es handelt es sich um ein hohles Gehäuse mit etwa rechteckigem Querschnitt. Das Gehäuse wird insbesondere aus zwei Kunststoffteilen gegossen, die dann miteinander verbunden werden, nachdem der Teststreifen in den Innenraum des Gehäuses eingelegt wurde. Auch die Ausführungsform der Fig. 1 und 2 zeigt den zur Gänze innerhalb des Gehäuses untergebrachten Teststreifen. Diesem Teststreifen ist der gesonderte Benetzungskörper als poröses Zusatz- und Kalibrierteil zugeordnet, welches in der Transportstellung durch eine Kappe abgedeckt ist. Nachdem das poröse Zusatzteil die vorgesehene Menge Urin aufgenommen hat, wird die Kappe wiederum mit dem Gehäuse verbunden, so daß nunmehr auch dieses Zusatzteil geschützt untergebracht ist. Das Gehäuse der Vorrichtung soll aus Kunststoff gefertigt werden. Auf jeden Fall handelt es sich um ein hohles Gehäuse, welches zumindest aus dampfundurchlässigem Material bestehen soll.

Aus der EP-A-0 362 809 ist eine Vorrichtung zur immunologischen Bestimmung von HCG im Urin als Schwangerschaftsfrühtest bekannt. Die Vorrichtung weist einen Teststreifen auf, der in einem Gehäuse untergebracht ist. Der Teststreifen weist die Form eines Stabes, eines kleinen Rohres oder eines Streifens auf und ist auf einem Träger aus Kunststoff, Papier od. dgl. aufgebaut. Der Teststreifen weist Urin weiterleitendes hydrophiles Material sowie Antikörper auf. Der Teststreifen besitzt keinen Benetzungsvorsprung, sondern ist ganz in dem hohlen Gehäuse aus Kunststoff untergebracht, das einen runden Querschnitt besitzt.

Der Erfindung liegt die Aufgabe zugrunde, eine Vorrichtung der eingangs beschriebenen Art bereitzustellen, die auch bei direkter Benetzung ein sicheres Ergebnis erbringt, sich in einfacher Weise ordnungsgemäß benutzen läßt und eine vergleichsweise erleichterte Entsorgung ermöglicht.

Erfindungsgemäß wird dies bei der Vorrichtung der eingangs beschriebenen Art dadurch erreicht, daß das Gehäuse eine flache, langgestreckte Gestalt besitzt, aus Pappe, Papier oder einem anderen lignozellulosehaltigen Rohstoff besteht und zwei Lagen aufweist, zwischen denen der Teststreifen aufgenommen ist, und daß der Umriß des flachen Gehäuses für eine sichere Handhabung breiter und länger als der Umriß des Teststreifens ausgebildet ist.

Die Erfindung geht von dem Gedanken aus, den Teststreifen nicht mehr in einem Gehäuse aus Kunststoff, sondern in einem aus Pappe, Papier oder einem anderen lignozellulosehaltigen Rohstoff unterzubringen und sich damit in deutlichen Gegensatz zu der im Stand der Technik vorherrschenden Entwicklungsrichtung zu setzen. Die Verwendung von Pappe für das Gehäuse erbringt nicht nur eine erleichterte Entsorgungsmöglichkeit und ähnliche Schutzfunktionen, wie sie auch ein Gehäuse aus Kunststoff erbringt. Zusätzlich vorteilhaft ist hieran, daß ein solches Gehäuse vergleichsweise dünn ausgebildet sein kann und daher nur einen geringen Raumbedarf erfordert. Gleichzeitig ist es aber möglich, das Gehäuse breit und lang auszubilden, so wie es für eine sichere, hygienische und zuverlässige Handhabung sinnvoll ist. Die beiden Lagen des Gehäuses umschließen bzw. nehmen zwischen sich den Teststreifen teilweise auf, so daß nur noch der Benetzungsvorsprung gegenüber dem Gehäuse vorsteht. Durch die vergleichsweise breitere und längere Gestaltung des Gehäuses aus Pappe, Papier oder einem anderen lignozellulosehaltigen Rohstoff eröffnet sich in einfacher Weise die Möglichkeit, das Gehäuse als Träger für Informationen, insbesondere solche, die die ordnungsgemäße Benutzung erleichtern, zu nutzen. Hierdurch wiederum kann die Notwendigkeit der Beifügung eines Beilagezettels entfallen oder zumindest stark darauf hingewirkt werden, daß bei der Benutzung Anwendungsfehler gemacht werden. Die Ausbildung des Gehäuses aus Pappe, Papier o. dgl. schafft gleichzeitig die Möglichkeit, die saugenden Eigenschaftes dieses Materials zur Unterstützung des Transportes entlang des Teststreifens zu nutzen. Diese saugenden Eigenschaften können auch dazu benutzt werden, um die negativen Eigenschaften von Fehlbenetzungen an nicht vorgesehener Stelle zu vermeiden bzw. in ihren negativen Folgen zu mindern.

Das Gehäuse kann den Benetzungsvorsprung teilweise überdecken, um durch Nutzung der saugenden Eigenschaften der Pappe, des Papiers o. dgl. den Transport des Urins in Längsrichtung des Teststreifens zu unterstützen bzw. beschleunigt in Gang zu bringen.

Das Gehäuse mit seinen beiden Lagen kann an dem dem Benetzungsvorsprung abgekehrten Ende zwei Schutzschenkel aufweisen, die auf den Benetzungsvorsprung so rückfaltbar sind, daß die rückgefalteten Schutzschenkel den Benetzungsvorsprung abdecken. Dies ist in verschiedener Hinsicht vorteilhaft. Einerseits schließen die rückgefalteten Schutzschenkel den Benetzungsvorsprung von beiden Seiten abdekkend ein, so daß der Benetzungsvorsprung vor mechanischer Beschädigung geschützt untergebracht ist. Gleichzeitig erhält damit die Vorrichtung einen gegenüber der Gebrauchslage verkleinerten Umriß, so daß sie platzsparend beispielsweise in einem Flachbeutel unterbringbar ist und so in Verkehr gebracht werden kann. Andererseits besitzt aber die Vorrichtung in der Gebrauchslage mit den aufgefalteten Schutzschenkeln eine relativ große Länge, so daß eine hygienische Anwendung auch bei Direktbenetzung möglich ist und ausreichend Raum für aufgedruckte Informationen betr. die Gebrauchsanwendung und die Ablesung des Ergebnisses geschaffen ist. Hierdurch werden Anwendungsfehler vermieden und die Sicherheit und Zuverlässigkeit bei der Durchführung des Testes erhöht.

Eine der Lagen des Gehäuses kann ein Fenster aufweisen, das so relativ zu dem aufgenommenen Teststreifen angeordnet ist, daß dieser durch das Fenster im Bereich eines Anzeigeabschnitts sichtbar ist. Ein solches Fenster läßt sich in einfacher Weise durch eine Ausstanzung bei der Herstellung des Gehäuses realisieren. Das Material des Gehäuses kann auf seiner nach außen gekehrten Oberfläche mit einer Beschichtung, Einfärbung o. dgl. versehen sein, die eine gewisse Abdichtwirkung der Oberfläche sicherstellt. Durch die Ausstanzung des Fensters werden jedoch die saugenden Schichten des Gehäuses freigelegt, so daß auch im Falle einer Fehlbenetzung des durch das Fenster sichtbaren Teststreifens der Urin durch die saugenden Eigenschaften des Gehäuses abgeleitet werden.

Es ist aber auch möglich, das Fenster mit einem abziehbaren Schutzstreifen abzudecken, so daß von vornherein die Gefahr einer Fehlbenetzung des Teststreifens gebannt ist. Der Schutzstreifen muß freilich abgezogen werden, um letztendlich das Ergebnis von dem Anzeigeabschnitt des Teststreifens ablesen zu können. In der Regel wird dieser abziehbare Schutzstreifen aus nicht-durchsichtigen Material bestehen, beispielsweise aus Folie, Papier o. dgl.. Es ist aber auch möglich, daß das Fenster mit einem durchsichtigen Schutzstreifen abgedeckt ist. In diesem Falle erübrigt sich ein Abziehvorgang. Der Teststreifen ist gänzlich geschützt in dem Gehäuse vorgesehen, mit Ausnahme des hervorragenden Benetzungsvorsprungs. Der Teststreifen kann insoweit nur an der vorgesehenen Stelle mit dem Urin in Kontakt gebracht werden.

Mindestens eine der beiden Lagen des Gehäuses können mit Informationen betr. die Anwendung der Vorrichtung versehen sein. Dies erbringt eine beachtliche Steigerung der Zuverlässigkeit, weil die Anwendungsinformationen während der Benutzung direkt auf der Vorrichtung sichtbar sind und nicht parallel zur Anwendung von einem Beilagezettel abgelesen werden müssen. Die bekannten Kunststoffgehäuse weisen solche Gebrauchsinformationen nicht auf. Hierzu steht einerseits der notwendige Platz nicht zur Verfügung, andererseits wäre ein Bedruckes solcher Kunststoffgehäuse ein zusätzlicher aufwendiger Arbeitsschritt. Wenn das Gehäuse aus Pappe, Papier o. dgl. besteht, ist ein Druckvorgang in einfacher Weise möglich.

Es besteht die Möglichkeit, beide Lagen des Gehäuses bereichsweise unterschiedlich saugend auszustatten, um damit unterschiedlichen Anforderungen zu genügen. Die unterschiedlich saugende Ausstattung kann sich sowohl auf die Längsrichtung der Vorrichtung als auch quer dazu beziehen. Es ist auch möglich, die beiden Lagen des Gehäuses bereichsweise unterschiedlich zu beschichten, um durch diese Maßnahme auf die saugenden Eigenschaften Einfluß zu nehmen.

Zur Vorrichtung gehört schließlich noch ein Flachbeutel, der zur Aufnahme der Vorrichtung mit rückgefalteten Schutzschenkeln dient. Damit kann die Vorrichtung platzsparend und mit kleinerem Umriß als in der Gebrauchslage im Transport zu der Anwenderin gelangen.

Die Vorrichtung wird anhand bevorzugter Ausführungsbeispiele weiter erläutert und beschrieben. Es zeigen:
- Figur 1: eine Draufsicht auf die Vorrichtung in einer ersten Ausführungsform,
- Figur 2: eine Seitenansicht der Vorrichtung gemäß Figur 1 in vergrößerter Höhendarstellung, um die einzelnen Lagen sichtbar zu machen,
- Figur 3: eine Draufsicht auf die eine Lage eines Gehäuses in einer zweiten Ausführungsform und
- Figur 4: eine Draufsicht auf die andere Lage des Gehäuses der Ausführungsform gemäß Figur 3.

Die in Figur 1 dargestellte Vorrichtung 1 weist ein Gehäuse 2 auf, welches aus Pappe, Papier oder einem anderen zellulosehaltigen Rohstoff besteht, und eine flache, langgestreckte Gestalt besitzt. Das Gehäuse 2 besteht aus zwei Lagen 3 und 4, die flach aufeinander liegen und zumindest bereichsweise miteinander verbunden sind. Eine solche Verbindungsstelle 5 kann sich über einen Teil des Randes des Gehäuses 2 erstrecken und/oder auch vom Rand eingeschlossene Bereiche aufweisen. Das Gehäuse 2 nimmt zwischen seinen beiden Lagen 3 und 4 einen Teststreifen 6 auf, der mit dem größten Teil seiner Länge zwischen den beiden Lagen 3 und 4 angeordnet und dort fixiert ist, während ein anderer Teil des Teststreifens 6 aus dem Gehäuse 2 herausschaut bzw. das Gehäuse 2 an seinem einen Ende überragt. Dieser Teil des Teststreifens 6 endet in einem Benetzungsvorsprung 7. Der Benetzungsvorsprung 7 endet an einer Markierung 8, die die maximale Eintauchtiefe anzeigt.

In Figur 1 ist eine Draufsicht auf die obere Lage 3 des Gehäuses 2, also auf die Vorderseite der Vorrichtung dargestellt. Die Lage 3 des Gehäuses 2 besitzt ein ausgestanztes Fenster 9, welches die Draufsicht auf einen Anzeigeabschnitt 10 des Teststreifens 6 ermöglicht. Auf dem Anzeigeabschnitt 10 sind im Falle des Vorliegens einer Schwangerschaft nach ordnungsgemäßer Durchführung des Testes zwei Kontrollstreifen 11 und 12 sichtbar, die meist in Form von Farbstreifen bzw. eines Farbumschlages an der betreffenden Stelle infolge der abgelaufenen Reaktion entstehen. Sind beide Kontrollstreifen 11 und 12 nach der Reaktion sichtbar, wurde das Hormon HCG festgestellt, und es liegt Schwangerschaft vor. Erscheint dagegen nach durchgeführter Reaktion allein der Kontrollstreifen 11, dann zeigt dies an, daß die Reaktionen ordnungsgemäß abgelaufen sind, sich die Vorrichtung in unbeschädigtem, ordnungsgemäßem Zustand befand und das Hormon HCG nicht festgestellt werden konnte. In diesem Falle liegt mit hoher Sicherheit keine Schwangerschaft vor.

Das Fenster 9 kann als Ausstanzung zunächst offen gestaltet sein, d. h. der Anzeigeabschnitt 10 liegt ungeschützt unterhalb dieses Fensters 9, so daß die Gefahr besteht, daß bei einer Direktbenetzung mit Urin die Vorrichtung 1 auch im Bereich dieses Fensters 9 versehentlich benetzt wird, obwohl für die Benetzung an sich nur der Benetzungsvorsprung 7 des Teststreifens 6 vorgesehen ist. Durch die saugenden Eigenschaften der Lage 3 des Gehäuses 2 aus Pappe, Papier o. dgl. wird jedoch dieser Urin beschleunigt von dem Anzeigeabschnitt 10 hinweggeführt, so daß damit nachteilige Auswirkungen vermieden werden. Noch besser ist es freilich, wenn das Fenster 9 durch einen Schutzstreifen 13 abgedeckt wird. Der Schutzstreifen 13 kann aus durchsichtigem Material bestehen, beispielsweise einer Klarsichtfolie, die in Form eines Abschnittes auf die Innenseite der Lage 3 geklebt ist, wie dies insbesondere aus Figur 2 erkennbar ist. Eine andere Möglichkeit besteht darin, einen abziehbaren Schutzstreifen 13 zu verwenden, der dann zweckmäßig auf die Außenseite der Lage 3 aufgeklebt ist und nach der Benetzung der Vorrichtung 1 mit Urin abgezogen wird, damit der Anzeigeabschnitt 10 des Teststreifens 6 sichtbar wird.

Die Lage 3 des Gehäuses 2 besitzt an ihrem dem Benetzungsvorsprung 7 des Teststreifens 6 abgekehrten Ende einen Schutzschenkel 14, der um eine Faltlinie 15 gegenüber dem übrigen Bereich 16 der Lage 3 gemäß Pfeil 17 um 180° in Richtung auf den Benetzungsvorsprung 7 des Teststreifens 6 rückfaltbar ist, so daß sein Umriß dann gemäß der strichpunktiert angedeuteten Linie den herausschauenden Teil des Teststreifens 6 abdeckt, überdeckt und schützt. Entsprechend dazu bildet auch ein Teil der Lage 4 des Gehäuses 2 einen Schutzschenkel 18, der um eine Faltlinie 19 in vergleichsweise gegenläufigem Faltsinn um 180° in Richtung auf den Benetzungsvorsprung 7 rückfaltbar ist, so daß sein Umriß dann ebenfalls mit der in Figur 1 strichpunktiert angedeuteten Linie zusammenfällt. Es ist erkennbar, daß diese rückgefaltete Stellung der beiden Schutzschenkel 14 und 18 für die Transportstellung vorgesehen ist, so daß damit der Benetzungsvorsprung 7 vor einer Beschädigung, Abknickung o. dgl. geschützt ist. Die gesamte Vorrichtung kann dann mit geringem Umriß innerhalb eines Flachbeutels 20 untergebracht werden, dessen Umriß zur Verdeutlichung mit einer doppelt gepunkteten-gestrichelten Linie 21 angedeutet ist.

Figur 2 zeigt die Darstellung der Schutzschenkel 14 und 18 des Gehäuses 2 in nicht zurückgefalteter Lage, so daß sich so die Vorrichtung 1 in der Gebrauchslage befindet. Wie ersichtlich besitzt das Gehäuse 2 damit in der Längsrichtung, also in Richtung des Verlaufes des Teststreifens 6, eine hinreichende Länge, wie sie für eine hygienische Anwendung sinnvoll ist. Während des Transportes wird dagegen ein kleinerer Umriß benötigt.

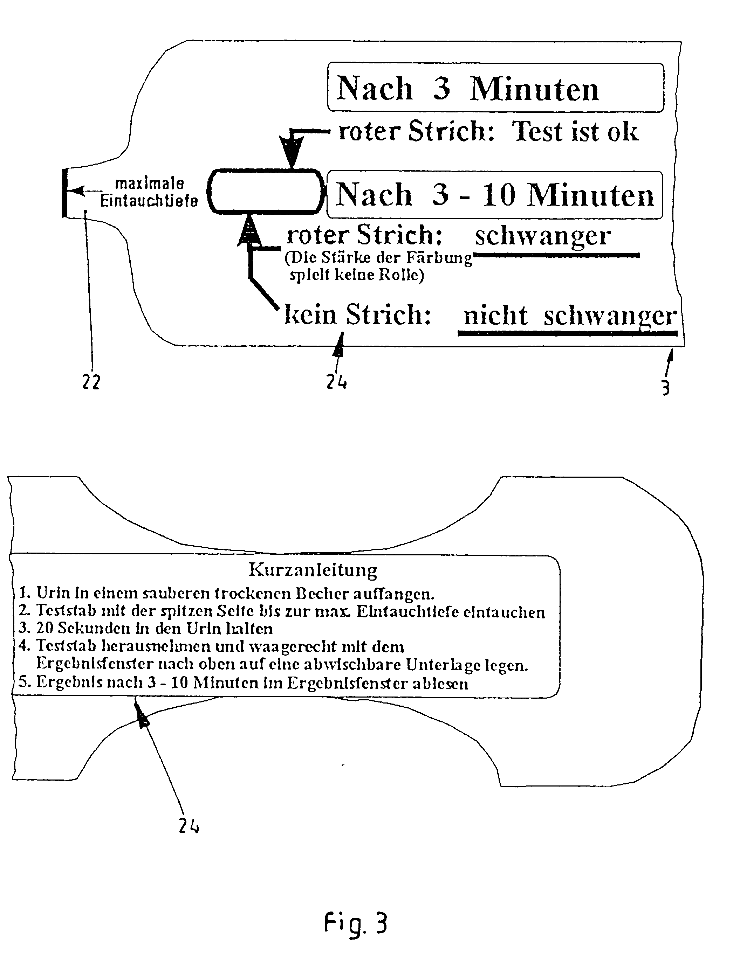

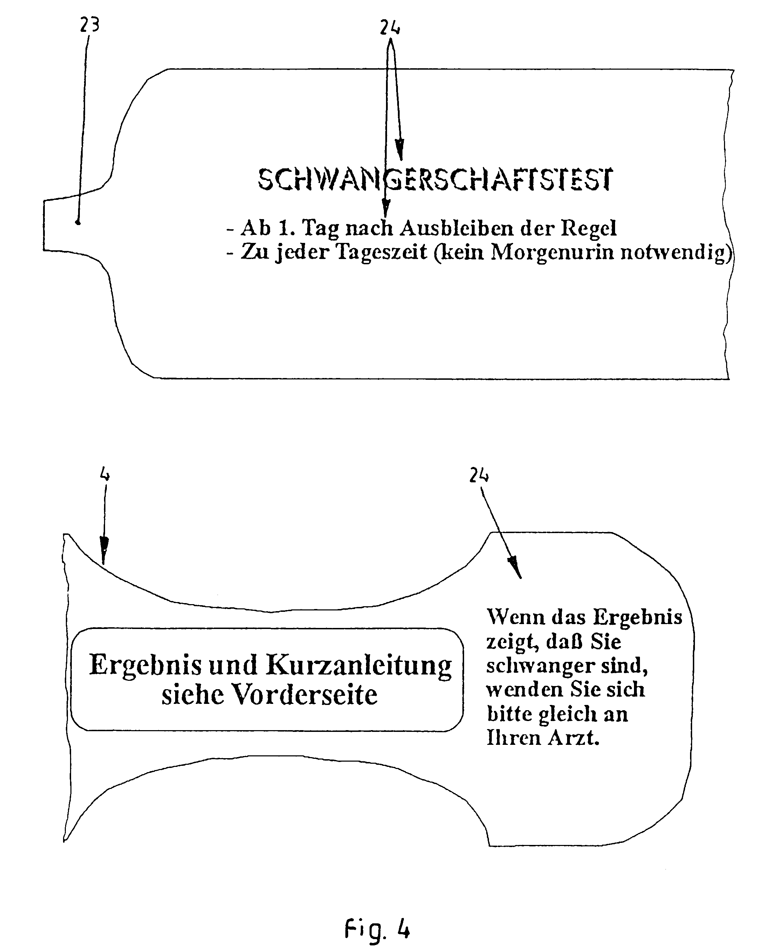

In den Figuren 3 und 4 sind die beiden Lagen 3 und 4, die zusammen das Gehäuse 2 bilden, dargestellt, und zwar bei einer anderen Ausführungsform. Die Lage 3 besitzt einen Vorsprung 22 und erstreckt sich damit bis in den Bereich des Benetzungsvorsprungs 7 des Teststreifens 6, der hier nicht dargestellt ist. Auch die letztlich die Rückseite bildende Lage 4 des Gehäuses 2 besitzt einen entsprechenden Vorsprung 23. Mit der Ausbildung der beiden Vorsprünge 22 und 23 wird ganz bewußt die Pappe, das Papier o. dgl. der beiden Lagen 3 und 4 des Gehäuses 2 in den Bereich des Benetzungsvorsprungs 7 des Teststreifens 6 gebracht, damit die saugenden Eigenschaften des Papiers für einen Anschub des Fließens des Urins in Richtung der Erstreckung des Teststreifens 6 benutzt werden kann. Die saugenden Eigenschaften des Papiers werden zusätzlich zu den saugenden Eigenschaften eines Glasfaserpolsters benutzt, welches sich mindestens im Bereich des Benetzungsvorsprungs 7 auf einem hier nicht näher dargestellten Träger des Teststreifens 6 befindet.

Wie anhand der Figuren 1, 3 und 4 erkennbar ist, sind die Lagen 3 und 4 und damit das Gehäuse 2 mit einem gegenüber dem Umriß des Teststreifens 6 vergleichsweise verlängerten und verbreiterten Umriß versehen, während die Höhe der Vorrichtung 1 sich nur aus der Höhe der Lagen 3 und 4 und der Höhe des Teststreifens 6 zusammensetzt. Damit entsteht ein Gehäuse 2, welches einerseits hinreichend groß ist, um eine einfache und hygienische Handhabung während der Durchführung des Testes zu ermöglichen. Andererseits ergeben sich damit auf den Lagen 3 und 4 hinreichend große Flächen, die mit einer aufgedruckten Information 24, wie sie anhand der Figuren 3 und 4 erkennbar ist, versehen ist. Die Flächen des Gehäuses 2 werden damit erstmals für die Wiedergabe einer Information 24 genutzt. Die Information 24 kann eine Gebrauchsanleitung auf einem Beilagezettel ersetzen oder doch zumindest hinsichtlich ihrer wesentlichen Punkte wiederholen, so daß damit die hinreichend genaue Anwendung bei der Durchführung des Schwangerschaftstestes gefördert und insoweit die Präzision und Sicherheit des Ergebnisses begünstigt wird. Sofern die Lagen 3 und 4 um Faltlinien 15 und 19 rückfaltbar sind, kann auch die Innenseite der Schutzschenkel 14 und 18 der Lagen 3 und 4 für das Aufbringen zusätzlicher Informationen genutzt werden.

Die beiden Lagen 3 und 4 des Gehäuses 2 können bereichsweise unterschiedlich saugend ausgebildet sein, beispielsweise mit einer Beschichtung versehen sein oder eine verdichtete Oberfläche besitzen.

### BEZUGSZEICHENLISTE

- 1 -: Vorrichtung
- 2 -: Gehäuse
- 3 -: Lage
- 4 -: Lage
- 5 -: Verbindungsstelle
- 6 -: Teststreifen
- 7 -: Benetzungsvorsprung
- 8 -: Markierung
- 9 -: Fenster
- 10 -: Anzeigeabschnitt
- 11 -: Kontrollstreifen
- 12 -: Kontrollstreifen
- 13 -: Schutzstreifen
- 14 -: Schutzschenkel
- 15 -: Faltlinie
- 16 -: Bereich
- 17 -: Pfeil
- 18 -: Schutzschenkel
- 19 -: Faltlinie
- 20 -: Flachbeutel
- 21 -: Linie
- 22 -: Vorsprung
- 23 -: Vorsprung
- 24 -: Information

## Patentansprüche

1. Vorrichtung zur Durchführung eines Schwangerschaftstests zur Feststellung des Hormons HCG im Urin, mit einem Teststreifen (6), der einen Träger mit einem Benetzungsvorsprung (7) aufweist und auf dem Urin weiterleitendes Material sowie Antikörper zur Anzeige entsprechender Reaktionen untergebracht sind, wobei der Teststreifen (6) mit dem bei Benutzung zumindest teilweise überstehenden Benetzungsvorsprung (7) in einem Gehäuse (2) angeordnet ist, **dadurch gekennzeichnet**, daß das Gehäuse (2) eine flache, langgestreckte Gestalt besitzt, aus Pappe, Papier oder einem anderen lignozellulosehaltigen Rohstoff besteht und zwei Lagen (3, 4) aufweist, zwischen denen der Teststreifen (6) aufgenommen ist, und daß der Umriß des flachen Gehäuses (2) für eine sichere Handhabung breiter und länger als der Umriß des Teststreifens (6) ausgebildet ist.

2. Vorrichtung nach Anspruch 1, **dadurch gekennzeichnet**, daß das Gehäuse (2) den Benetzungsvorsprung (7) teilweise überdeckt.

3. Vorrichtung nach Anspruch 1, **dadurch gekennzeichnet**, daß das Gehäuse (2) mit seinen beiden Lagen (3, 4) an dem dem Benetzungsvorsprung (7) abgekehrten Ende zwei Schutzschenkel (14, 18) aufweist, die auf den Benetzungsvorsprung (7) so rückfaltbar sind, daß die rückgefalteten Schutzschenkel (14, 18) den Benetzungsvorsprung (7) abdecken.

4. Vorrichtung nach Anspruch 1, **dadurch gekennzeichnet**, daß eine der Lagen (3) des Gehäuses (2) ein Fenster (9) aufweist, das so relativ zu dem aufgenommenen Teststreifen (6) angeordnet ist, daß dieser durch das Fenster (9) im Bereich eines Anzeigeabschnitts (10) sichtbar ist.

5. Vorrichtung nach Anspruch 4, **dadurch gekennzeichnet**, daß das Fenster (9) mit einem abziehbaren Schutzstreifen (13) abgedeckt ist.

6. Vorrichtung nach Anspruch 4, **dadurch gekennzeichnet**, daß das Fenster (9) mit einem durchsichtigen Schutzstreifen (13) abgedeckt ist.

7. Vorrichtung nach Anspruch 1, **dadurch gekennzeichnet**, daß mindestens eine der beiden Lagen (3 oder 4) des Gehäuses (2) mit Informationen (24) betreffend die Anwendung der Vorrichtung (1) versehen ist.

8. Vorrichtung nach Anspruch 1, **dadurch gekennzeichnet**, daß die beiden Lagen (3, 4) des Gehäuses (2) bereichsweise unterschiedlich saugend ausgestattet sind.

9. Vorrichtung nach Anspruch 8, **dadurch gekennzeichnet**, daß die beiden Lagen (3, 4) des Gehäuses (2) bereichsweise unterschiedlich beschichtet sind.

10. Vorrichtung nach Anspruch 3, **dadurch gekennzeichnet**, daß ein Flachbeutel (20) vorgesehen ist, der zur Aufnahme der Vorrichtung (1) mit rückgefalteten Schutzschenkeln (14, 18) dient.

## Claims

1. Device to execute a pregnancy test to determine the HCG hormone in urine, comprising a test strip (6) including a carrier with a wetting protruding element (7), material transmitting urine and antibodies for indicating corresponding reactions being arranged on the test strip (6), the test strip (6) with the wetting protruding element (7) at least partially projecting during use being arranged in a housing (2), **characterized in** that the housing (2) has a flat, elongated shape, the housing (2) is made of paperboard, paper or another resource material containing lignocellulose and the housing (2) includes two layers (3, 4) between which the test strip (6) is arranged, and that the outline of the flat housing (2) is designed to be wider and longer than the outline of the test strip (6) for a secure handling.

2. Device according to claim 1, **characterized in** that the housing (2) partially covers the wetting protruding element (7).

3. Device according to claim 1, **characterized in** that the housing (2) with its two layers (3, 4) includes two protection legs (14, 18) being arranged at the end facing away from the wetting protruding element (7), the two protection legs (14, 18) being foldable back onto the wetting protruding element (7), so that the two protection legs (14, 18) cover the wetting protruding element (7).

4. Device according to claim 1, **characterized in** that one of the layers (3) of the housing (2) includes a window (9) being arranged with respect to the test strip (6), so that the test strip (6) is visible through the window (9) in the region of an indicating section (10).

5. Device according to claim 4, **characterized in** that the window (9) is covered by a removable protection strip (13).

6. Device according to claim 4, **characterized in** that the window (9) is covered by a transparent protection strip (13).

7. Device according to claim 1, **characterized in** that at least one of the two layers (3 or 4) of the housing (2) is provided with information (24) concerning die application of the device (1).

8. Device according to claim 1, **characterized in** that the two layers (3, 4) of the housing (2) are designed to suck differently in certain regions.

9. Device according to claim 1, **characterized in** that the two layers (3, 4) of the housing (2) are differently coated in certain regions.

10. Device according to claim 3, **characterized in** that a flat bag (20) serving to contain the device (1) with the folded back protection legs (14, 18) is provided.

## Revendications

1. Dispositif pour exécuter une épreuve de grossesse pour la détection de l'hormone HCG dans l'urine, avec une bandelette de test (6) qui présente un support pour une protubérance d'imbibition (7), et dans lequel sont disposés un matériau transmetteur d'urine ainsi que des anticorps pour l'affichage de réactions adéquates, où la bandelette de test (6) avec la protubérance d'imbibition (7), qui lors de l'utilisation est au moins partiellement en saillie, est contenue dans un étui (2), caractérisé en ce que l'étui (2) a une configuration plate allongée, est fabriqué en carton, en papier ou en un autre matériau contenant une matière ligneuse ou à base de cellulose et présente deux couches (3, 4) entre lesquelles est située la bandelette de test (5), et en ce que le contour de l'étui plat (2) est, aux fins d'un maniement en toute sécurité, plus large et plus long que le contour de la bandelette de test (6).

2. Dispositif suivant la revendication 1, caractérisé en ce que l'étui (2) recouvre partiellement la protubérance d'imbibition (7).

3. Dispositif suivant la revendication 1, caractérisé en ce qu'à l'extrémité opposée à la protubérance d'imbibition (7), l'étui (2) dotée de ses deux couches (3, 4) présente deux languettes de protection (14, 18) qui peuvent être rabattues sur la protubérance d'imbibition (7) de manière telle que les languettes de protection rabattues (14, 18) recouvrent la protubérance d'imbibition (7).

4. Dispositif suivant la revendication 1, caractérisé en ce qu'une des deux couches (3) de l'étui (2) présente un regard (9), qui par rapport à la bandelette de test (6) contenue dans l'étui est disposé de manière telle que cette bandelette est dans sa zone d'affichage (10) visible au travers le regard (9).

5. Dispositif suivant la revendication 4, caractérisé en ce que le regard (9) est recouvert d'une bande de protection (13) qui peut être retirée.

6. Dispositif suivant la revendication 4, caractérisé en ce que le regard (9) est recouvert d'une bande de protection transparente (13).

7. Dispositif suivant la revendication 1, caractérisé en ce qu'au moins une des deux couches (3 ou 4) de l'étui (2) est dotée d'informations (24) relatives à l'utilisation du dispositif (1).

8. Dispositif suivant la revendication 1, caractérisé en ce que les deux couches (3, 4) de l'étui (2) ont suivant les zones des pouvoirs d'absorption différents.

9. Dispositif suivant la revendication 8, caractérisé en ce que les deux couches (3, 4) de l'étui (2) sont suivant les zones revêtues de manière différente.

10. Dispositif suivant la revendication 3, caractérisé en ce qu'un sachet plat (20) est prévu, qui sert à la réception du dispositif (1) avec les languettes de protection (14, 18) rabattues vers l'arrière.
